# EUROPÄISCHE PATENTSCHRIFT

(11) **EP 2 179 761 B1**
(45) Veröffentlichungstag und Bekanntmachung des Hinweises auf die Patenterteilung: **14.12.2011**
(21) Anmeldenummer: 09013244.0
(22) Anmeldetag: 20.10.2009
(51) Int. Cl.: A61M 16/04

(54) **Trachealtubus**
Tracheal tube
Tube trachéen

(30) Priorität: 21.10.2008 DE 102008052438
(43) Veröffentlichungstag der Anmeldung: 28.04.2010
(73) Patentinhaber: Willy Rüsch GmbH, 71394 Kernen-Rommelshausen (DE)
(72) Erfinder: Köhler, Dieter, Prof. Dr., 57392 Schmallenberg (DE)
(74) Vertreter: Grünecker, Kinkeldey, Stockmair & Schwanhäusser

(56) Entgegenhaltungen:
- EP-A1- 1 329 239
- WO-A1-86/02564
- WO-A1-2005/009522
- US-A- 4 280 492
- US-A- 4 495 948
- US-A1- 2007 113 857
- US-B1- 6 722 367

## Beschreibung

Die Erfindung bezieht sich auf einen Trachealtubus zur Beatmung von Patienten mittels eines druckgesteuerten Respirators mit einem Beatmungsschlauch, der zumindest teilweise in die Trachea einführbar ist, mit einer Dichtungsmanschette, die an dem in die Trachea einführbaren Teil um den Beatmungsschlauch herum angeordnet ist, mit mindestens einer Belüftungskanüle, die in die Dichtungsmanschette mündet, und mit einer Absaugeinrichtung.

Trachealtubusse zur Beatmung von Patienten bei unzureichender oder ausgesetzter Atmung sind in der Technik in vielfältigen Formen und Ausführungen bekannt und dienen dazu respiratorisches Gas in die Lungen eines Patienten zu bringen. Dazu wird der Tubus in die Trachea (Luftröhre) des Patienten endotracheal oder tracheostomatisch eingeführt. An dem distalen in die Trachea eingeführten Ende des Beatmungsschlauchs ist üblicherweise eine Dichtungsmanschette (Cuff) vorgesehen, die dazu ausgelegt ist, die Abdichtung zwischen dem Beatmungsschlauch und der Trachea sicherzustellen. Dabei wird die Dichtungsmanschette nicht im aufgeblasenen Zustand in die Trachea eingeführt, sondern erst mit Luft gefüllt, sobald sich der Beatmungsschlauch an seiner vorgesehenen Position befindet. Für eine einfache Anpassung an unterschiedliche Durchmesser und eine sichere Abdichtung sind die Dichtungsmanschetten solcher Tracheltubusse oval oder kugelig ausgebildet. Die aufgeblasene Dichtungsmanschette bewirkt, dass das respiratorische Gas bis in die unteren Luftwege eindringen kann, um so eine ausreichende Sauerstoffversorgung des Patienten sicherzustellen. Darüber hinaus verhindert die Dichtungsmanschette insbesondere bei tief bewusstlosen oder atemgelähmten Patienten die Aspiration von aus dem Rachen und der Mundhöhle zurückströmenden Flüssigkeiten, die beim Eindringen in die Lunge zu schwerwiegenden Komplikationen führen können, z.B. einer Aspirationspneunomie.

Herkömmlicherweise wird die Dichtungsmanschette unabhängig von der Beatmungsluft mit Gas gefüllt, wobei auch der Druck beim Aufblasen der Dichtungsmanschette unabhängig von dem Druck des in die Lunge des Patienten inspirierten respiratorischen Gases ist. Eine derart aufgeblähte Dichtungsmanschette drückt dann für eine kontinuierliche Abdichtung zwischen Beatmungsschlauch und der Trachea ständig gegen die Innenwände der Trachea. Auch wenn der Aufblasdruck der Dichtungsmanschette auf einen niedrigst möglichen Wert für eine ausreichende Abdichtung begrenzt wird, wirkt der Anlagedruck der Dichtungsmanschette gegen die Wände der Trachea für diese traumatisch. Insbesondere bei einem längerfristigen Einsatz eines Trachealtubus verursacht der Druck auf die Trachea und die damit verbundenen Durchblutungsstörungen Gewebsnekrosen, Trachealstenosen oder Druckulceras, die ebenso wie zurückbleibende Narben im Bereich der Dichtungsmanschette schwerwiegende und langwierige Nachbehandlungen erfordern und zu lebenslanger Ateminsuffizienz führen können.

Um die Probleme zu reduzieren, die sich bei der notwendigen Abdichtung der Trachea durch den Anpressdruck der Dichtungsmanschette an die Tracheawand ergeben, wurden im Stand der Technik eine Reihe von Entwicklungen vorgenommen, die im Wesentlichen auf eine geeignete Druckbegrenzung der Dichtungsmanschette gerichtet waren. Beispielsweise beschreiben die Druckschriften DE 91 04 637 U1, DE 33 21 155 C2 und US 5,487,383 Vorrichtungen, bei denen der Druck in der Dichtungsmanschette gemessen wird und durch eine entsprechende Steuereinrichtung auf den niedrigst möglichsten Wert begrenzt wird. Demgegenüber zeigt die Druckschrift WO 91/10465 A1 einen Tubus mit einem Luftreservoir zum Überwachen des Drucks der Abdichtungsmanschette, wobei sich entsprechend dem Druck in der Manschette mittels einer elastischen Membran die optischen Eigenschaften einer semitransparenten Platte ändern. Eine weitere Anzeigevorrichtung für den Druck in der aufblasbaren Dichtungsmanschette wird in der EP 231 564 A1 beschrieben, bei der der Druck ebenfalls mittels einer flexiblen Membran angezeigt wird.

Im Hinblick auf die Abdichtung gegenüber einer Aspiration müssen die sich oralwärts der Dichtungsmanschette im subglottischen Bereich der Trachea, der Bereich der Luftröhre zwischen der Druckmanschette des Trachealtubus und den Stimmbändern, ansammelnde Absonderungen und Sekrete nach Bedarf mittels eines üblicherweise oral eingeführten Katheters abgesaugt werden. Darüber hinaus gibt es auch Trachealtubusse mit einem parallel zum Beatmungsschlauch geführten Absaugkatheter, beispielsweise die Tubusse aus der DE 44 45 428 C1 und der DE 195 33 615 C1, mit dem die subglottischen Absonderungen und Sekrete bei Bedarf abgesaugt werden können. Lungenwärts der Dichtungsmanschette soll das Entstehen von Sekret bzw. das Verbleiben von Sekret in der Trachea möglichst vermieden werden. Deshalb wird das respiratorische Atemgas üblicherweise befeuchtet und angewärmt. Neben der Verringerung der Sekretproduktion wird durch die Befeuchtung der Atemluft im bronchialen Bereich entstehendes Sekret möglichst flüssig gehalten, so dass das Sekret zum Teil bei der Exspiration mit dem Luftstrom herausgeführt werden kann. Sekret, das sich lungenwärts der Druckmanschette in der Trachea und dem Beatmungsschlauch ansammelt, muss über spezielle in den Beatmungsschlauch einzuführende Absaugkanülen abgesaugt werden, um die Infektionsgefahr zu verringern. Diese regelmäßig durchzuführende Bronchialtoilette wird von den Patienten als unangenehm und belastend empfunden.

Ein weiteres Konzept den traumatischen Druck der Dichtungsmanschette gegen die Wände der Trachea zu reduzieren, wird in der US 3,707,151 beschrieben. Bei diesem Trachealtubus wird die Dichtungsmanschette über Öffnungen im Beatmungsschlauch auf Höhe der Manschette mit dem auch in die Lungen des Patienten einströmenden respiratorischen Gas gefüllt. So bläst sich die Dichtungsmanschette auf und lässt den Druck im Rhythmus des Ausblasens des respiratorischen Gases wieder ab. Dadurch wirkt der Anpressdruck, mit dem die Dichtungsmanschette gegen die Wände der Trachea abdichtet, nicht ununterbrochen, sondern zyklisch. Ferner entspricht der Druck in der Manschette dem des respiratorischen Gases, dessen Höhe als möglichst nicht traumatisch für die Lungen des Patienten ausgewählt wird. Derartige Trachealtubusse sind üblicherweise nur für den Betrieb mit volumengesteuerten Respiratoren geeignet, die heutzutage fast vollständig von den überlegenden druckgesteuerten Respiratoren verdrängt wurden. Obgleich die Abdichtung durch die Dichtungsmanschette nur zyklisch wirkt, erfolgt nach dem Aufblasen der Dichtungsmanschette während der Inspiration des respiratorischen Gases in die Lungen eine Abdichtung, die insgesamt den Rückfluss des respiratorischen Gases oralwärts vermeidet. Ein Zusammenfallen der Druckmanschette wird durch eine spezielle Geometrie der Einlassöffnungen (in die Dichtungsmanschette) durch die Wand des Beatmungsschlauchs oder durch geeignete Ventileinrichtungen verhindert. Ein weiterer Trachealtubus mit einer ventilierten Dichtungsmanschette zeigt die EP 1 329 239 A1, wobei die Dichtungsmanschette durch einen Schlitz im Beatmungsschlauch mit dem Inneren des Beatmungsschlauchs verbunden ist. Dabei entsteht während der Expiration zwischen der Dichtungsmanschette und den Wänden der Trachea ein Spalt, durch den ein Teil des ausgeatmeten respiratorischen Gases an den Stimmbändern des Patienten vorbei ausströmt. Die Druckschrift EP 640 355 B1 beschreibt eine Fortbildung eines solchen Trachealtubus mit offener Dichtungsmanschette, bei der die Dichtungsmanschette selbst oder ein zweiter, die Dichtungsmanschette umgebender Druckraum mittels einer kapillaren Leitung direkt mit dem Respirator verbunden ist. Auch die Druckschrift WO 2005/009522 A1 offenbart einen Trachealtubus mit einer offenen Dichtungsmanschette, die über eine Kanüle in der Wand des Beatmungsschlauchs mit dem Respirator verbunden ist und im Rhythmus der Beatmung des Patienten aufgeblasen und entlastet wird.

Eine weitere bekannte Ausführung eines Trachealtubus mit einer zum Beatmungsschlauch offenen Dichtungsmanschette beschreibt die DD 68 597. Dieser Trachealtubus ist mit einem separaten Absaugkanal versehen. Beim Einströmen der respiratorischen Atemluft über den Beatmungsschlauch wird die am Ende dieses Kanals angeordnete Dichtungsmanschette aufgeblasen, da ein Teil des respiratorischen Gases zwangsweise über mehrere Öffnungen durch die Dichtungsmanschette geführt wird, wobei die Öffnungen durch eine Einschnürung in dem Beatmungsschlauch beabstandet sind. Diese Einschnürung bewirkt auch bei der Exspiration über den Beatmungsschlauch ein Aufblähen der Dichtungsmanschette und ein Anliegen an die Wände der Trachea. Über den Absaugkanal kann während der Exspiration oder in gewünschten Abständen gleichzeitig Luft und Sekret aus dem subglottischen Bereich abgesaugt werden. Trachealtubusse mit zum Beatmungsschlauch offenen Dichtungsmanschetten benötigen zum Aufblasen der Manschette den erhöhten Druck volumengesteuerter Respiratoren. Dabei liegt dieser Druck im aufgeblasenen Zustand der Dichtungsmanschette dann an der Trachea an und führt zu traumatischen Schäden, obwohl die Manschette nur während der Inspiration mit hohem Druck an der Trachea anliegt. Auch ist bei einfachen Ausführungen weiter die Gefahr der Aspiration gegeben.

Eine weitere Ausführungsform eines Trachealtubus zeigt die EP 152 694 B1. Bei diesem Trachealtubus wird die Dichtungsmanschette in üblicher Weise mit einem Druck beaufschlagt, der bei der geringsten Ausdehnung der Trachea eine Abdichtung gegenüber deren Wänden erzielt, wodurch auch beim Ausatmen eine Abdichtung gegenüber der Tracheawand sichergestellt ist und eine Aspiration verhindert wird. Diese Dichtungsmanschette ist von einem weiteren Ballon umgeben, der entweder ebenfalls unter einem Vordruck mit Gas gefüllt ist oder der sich über eine separate Leitung während der Inspiration mit respiratorischen Gas füllen kann, um die sich bei der Inspiration ausdehnende Trachea entsprechend dem Atemrhytmus abzudichten. Diese Form eines Trachealtubus ermöglicht eine ständige Abdichtung gegenüber der Wand der Trachea und verhindert so eine Aspiration, wobei auch bei der geringsten Ausdehnung der Trachea während der Exspiration ständig ein Druck auf die Wände der Trachea ausgeübt wird. Auch wenn dieser Druck auf die jeweilige Ausdehnung der Trachea angepasst ist, werden die Wände der Trachea trotz des komplexen konstruktiven Aufbaus weiterhin belastet.

Die Druckschrift WO 86/002564 A1 beschreibt einen weiteren Trachealtubus mit einer Dichtungsmanschette für eine kontinuierliche Abdichtung zwischen dem Beatmungsschlauch und der Trachea. Zusätzlich zu einem ersten Beatmungskanal ist in diesem Trachealtubus parallel ein zweiter Sprachkanal vorgesehen, der mit einem Stimmgenerator verbunden ist und über eine Reihe von Löchern in der Wand des Beatmungsschlauchs dem Rachenraum des Patienten Töne zum Sprechen zuführt.

Obwohl die in der Technik bekannten Trachealtubusse mit druckgesteuerten Dichtungsmanschetten zur Einstellung des minimal notwendigen Dichtungsdrucks als auch die Trachealtubusse mit zum Beatmungsschlauch offenen Dichtungsmanschetten die traumatischen Probleme der Trachea durch den Auflagedruck der Dichtungsmanschette verbessert haben, existiert bisher keine optimale Lösung dieses Problems. Zum Einen gibt es auch bei druckgesteuerten Dichtungsmanschetten weiterhin Druckschäden, zum Anderen ist bei offenen Dichtungsmanschetten die Infektionsgefahr deutlich erhöht, da sich sowohl in den Öffnungen als auch in der Dichtungsmanschette selbst Sekret sammeln kann. Die Aufgabe der vorliegenden Erfindung liegt daher darin, einen möglichst einfachen Trachealtubus bereitzustellen, der die im Stand der Technik bekannten Probleme vermeidet, insbesondere traumatische Druckschäden an der Trachea und die Infektionsgefahr.

Erfindungsgemäß wird dies bei einem gattungsgemäßen Trachealtubus gelöst, in dem die mindestens eine Belüftungskanüle mit ihrem der Dichtungsmanschette abgewandten Ende in den Beatmungsschlauch mündet, wobei die Manteldichtungsfläche der Dichtungsmanschette größer als die lungenwärtige ringförmige Stirnfläche der Dichtungsmanschette ist und die Absaugeinrichtung bevorzugt oralwärts in der Dichtungsmanschette absaugend angeordnet ist. Darüber hinaus ist ein Sprachventil vorgesehen, wobei das Sprachventil an dem nicht in die Trachea einführbaren Teil des Beatmungsschlauchs angeordnet ist und die mindestens eine Belüftungskanüle auf der der Dichtungsmanschette abgewandten Seite des Sprachventils in den Beatmungsschlauch mündet.

Ein derartiger erfindungsgemäßer Trachealtubus kann in jeweils geeigneten Ausbildungen sowohl als Endotrachealtubus, als auch für die Verwendung bei einem Tracheostoma eingesetzt werden, wobei der in die Trachea einführbare Teil des Beatmungsschlauchs entweder oral oder über das Tracheostoma eingeführt wird. Der Druck, mit dem sich die Dichtungsmanschette bei der Inspiration nach einer kurzen Verzögerungszeit aufbläst, entspricht dem Druck der vom Respirator bereitgestellten Atemluft. Während bei der Inspiration das gesamte respiratorische Gas dem Patienten über den Beatmungsschlauch zugeführt wird, kann bei der Exspiration ein Teil der Atemluft an der Dichtungsmanschette vorbei oralwärts ausgeatmet werden, da die Dichtungsmanschette des erfindungsgemäßen Trachealtubus im Rhythmus der druckgesteuerten Beatmung pulsiert. Als Beatmungsschlauch ist dabei das gesamte den Respirator mit der Lunge des Patienten verbindende Gebilde zu verstehen. Der während der Inspiration aufgeblasene und an der Wand der Trachea anliegende Dichtungsballon kollabiert nach dem Einsetzen der Exspiration zumindest teilweise. Das Zusammenfallen der Dichtungsmanschette wird nach dem Ende der Inspiration zunächst durch den Druckabfall in dem Beatmungsschlauch und in der fluidmäßig direkt damit verbundenen Dichtungsmanschette eingeleitet, wobei die während der Exspiration durch den Beatmungsschlauch zurückströmende Atemluft über die Belüftungskanüle Luft aus der Dichtungsmanschette abzieht. Zusätzlich unterstützt der Druck der Atemluft in der Lunge gegen die ringförmige Stirnfläche der Dichtungsmanschette das Zusammenfallen oder teilweise Kollabieren der Dichtungsmanschette. Sobald die Dichtungsmanschette zumindestens teilweise kollabiert ist, kann ein Teil des Exspirations-Luftstroms an der Dichtungsmanschette vorbei oralwärts durch die Trachea ausgeatmet werden, wodurch zusätzlich das Zusammenfallen der Dichtungsmanschette unterstützt wird. Der oralwärts an der Dichtungsmanschette vorbeiströmende Teil des Exspirations-Luftstroms kann bronchial entstehendes Sekret an der Dichtungsmanschette vorbei, aber unter Umständen auch andere im subglottischen Bereich der Trachea vorliegende Flüssigkeiten, oralwärts transportieren. Da die Inspiration vom respiratorischem Gas ausschließlich über den Beatmungsschlauch erfolgt, ist die Gefahr der Rückströmung von bronchialem Sekret und der Aspiration gering. Das Pulsieren oder Ventilieren der Dichtungsmanschette bei dem hier vorliegenden Trachealtubus wirkt somit nicht nur traumatischen Druckschäden an der Trachea entgegen, sondern reduziert auch die Infektions- und Aspirationsgefahr durch bronchiales Sekret, da dieses bei der Exspiration aus dem unteren Bereich der Trachea vorbei an der Dichtungsmanschette oralwärts transportiert wird, während die Inspiration ausschließlich über den Beatmungsschlauch erfolgt.

Das Sprachventil verhindert beim Ausatmen durch einen Ventilmechanismus das Ausströmen von Atemluft durch den Beatmungsschlauch. Die bei der Inspiration zugeführte Atemluft kann somit vollständig bei der Exspiration zur Stimmüberzeugung über die Stimmbänder geleitet werden. Sprachventile werden insbesondere zur Stärkung der Atemmuskulatur, zur therapeutischen Spontanatmung in regelmäßigen Abständen sowie zur Entwöhnung vom Respirator nach einer Langzeitbeatmung eingesetzt. Die Anordnung des Sprachventils zwischen der Dichtungsmanschette und dem Anschluss der einen oder mehrerer Belüftungskanülen an den Beatmungsschlauch, und damit üblicherweise an dem nicht in die Trachea einführbaren Teil des Beatmungsschlauchs, verhindert ein Blockieren der Dichtungsmanschette im aufgeblähten abgedichteten Zustand. Eine falsche Anordnung des Sprachventils jenseits des Anschlusses der Belüftungskanüle an den Beatmungsschlauch kann zu einem Zustand führen, in dem der Patient zwar einatmen bzw. beatmet werden kann, jedoch nicht ausatmen kann, was zunächst zu einer Überblähung der Lunge und Luftnot mit Panikzuständen aber schlussendlich auch zum Tod des Patienten führen kann. Um eine solche gefährliche Blockage der Dichtungsmanschette zu verhindern sind vorzugsweise am Sprachventil und dem Anschlussende des Beatmungsschlauchs und/oder der Anordnung der mindestens einen Belüftungskanüle an dem Beatmungsschlauch besondere Anschlüsse vorzusehen, um eine falsche Anordnung des Sprachventils am äußeren Ende des Beatmungsschlauchs zu verhindern. Alternativ kann am Sprachventil auch ein Bypass vorgesehen sein, der über eine Verbindung zur Belüftungskanüle die Entlüftung der Dichtungsmanschette gewährleistet.

Die Manteldichtungsfläche der Dichtungsmanschette ist der Teil der Außenwand der Dichtungsmanschette der im aufgeblasenen Zustand an der Wand der Trachea anliegt und der bei der Inspiration eine Abdichtung des Beatmungsschlauchs gegenüber der Trachea erreicht. Dabei muss die Manteldichtungsfläche größer als die lungenwärtige ringförmige Stirnfläche zwischen Beatmungsschlauch und Trachea sein, da ansonsten der durch die Belüftungskanüle hergestellte Druckausgleich, bzw. das Druckgleichgewicht zwischen der Dichtungsmanschette und der Lunge zu einem Ablösen der Manteldichtungsfläche von der Trachea führen würde. Dadurch kann dann ein Teil des bei der Inspiration eingeströmten respiratorischen Gases oralwärts an der Dichtungsmanschette vorbei entweichen. Die notwendige Interaktion zwischen der Dichtungsmanschette und der Belüftungskanüle und gegenüber dem Beatmungsschlauch und dem darin verlaufenden Atemluftstrom ist aufeinander abgestimmt, so dass sich der Druck in der Dichtungsmanschette bei der Inspiration ohne Verzögerung aufbaut und bei der Exspiration so weit und so schnell abbaut, um einen Teil des Exspirations-Luftstroms zusammen mit bronchialem Sekret an der Dichtungsmanschette vorbei oralwärts zu transportieren.

Durch den bei der Exspiration an der Dichtungsmanschette vorbei strömenden Teil des Atemluftstroms können die mit einem erfindungsgemäßen Trachealtubus intubierten Patienten bei der Exspiration sprechen, da der oralwärts ausgeatmete Atemluftstrom auch an den Stimmbändern vorbei strömt. Dies wird von den intubierten Patienten als sehr angenehm empfunden, da so dem Pflegepersonal leichter Bedürfnisse und Wünsche mitgeteilt werden können. Darüber hinaus reduziert der bei der Exspiration oralwärts gerichtete Teil des Atemstroms die Aspirationsgefahr, da der oralwärts gerichtete Luftstrom zum Einen bereits das Zurückfließen von Flüssigkeiten aus dem Rachenraum in die Trachea verringert und zum Anderen in der Trachea befindliche Flüssigkeiten zumindest zum Teil heraustransportiert.

Das Pulsieren der Dichtungsmanschette ermöglicht den Transport von bronchialem Sekret aus dem unteren Bereich der Trachea an der Dichtungsmanschette vorbei in den subglottischen Bereich, weshalb es bei dem erfindungsgemäßen Trachealtubus nicht länger notwendig ist, die Sekretproduktion von intubierten Patienten zu reduzieren, beispielsweise durch eine starke Befeuchtung und Vorwärmung des respiratorischen Gases oder medikamentös zu unterbinden. Durch das Ventilieren der Dichtungsmanschette wird so in den subglottischen Bereich der Trachea bronchiales Sekret gefördert. Die oralwärts der Dichtungsmanschette absaugend angeordnete Absaugeinrichtung des Trachealtubus ermöglicht es, dieses Sekret abzusaugen. Im Gegensatz zum Stand der Technik muss mit dem erfindungsgemäßen Trachealtubus die Entstehung und der oralwärtige Transport von Sekret nicht vermieden, bzw. nur im Moment der Absaugung zugelassen werden, sondern ist uneingeschränkt möglich.

Eine zweckmäßige Ausführungsform sieht vor, dass die Innenwand der um den Beatmungsschlauch herum angeordneten Dichtungsmanschette vollständig an dem Beatmungsschlauch anliegt oder von dem Beatmungsschlauch ausgebildet ist. Eine derartige Ausführung der Innenwand der Dichtungsmanschette ermöglicht eine einfache kostengünstige Konstruktion und vermeidet gleichzeitig hygienische Probleme sowie Infektionen, die durch ein Ein- oder Anlagern von Sekret entstehen können.

Für einen einfachen Aufbau des erfindungsgemäßen Trachealtubus kann das Volumen der Dichtungsmanschette zwischen dem Beatmungsschlauch und der Mantelfläche ungeteilt sein. Die Ausgestaltung der Dichtungsmanschette mit nur einer einzigen ungeteilten luftgefüllten Kammer ermöglicht ein sicheres Ventilieren bzw. Pulsieren der Dichtungsmanschette, so dass das Zusammenfallen oder Teilkollabieren der Dichtungsmanschette bei der Exspiration einen ausreichenden Spalt zwischen Trachea und Manschette erzeugt. Damit wird dann auch ein ausreichender Teil-Atemluftstrom für den oralwärtigen Sekrettransport bereitgestellt. Das ungeteilte Volumen der Dichtungsmanschette ermöglicht es so, das Funktionsprinzip der Dichtungsmanschette optimal umzusetzen.

Eine weitere Ausbildung sieht vor, dass die Dichtungsmanschette in dem in die Trachea einführbaren Teil gegenüber dem Beatmungsschlauch geschlossen, d.h. öffnungsfrei ausgebildet ist. Eine solche fluiddichte geschlossene Ausführung der Dichtungsmanschette verhindert einen Sekretstau oder -ablagerungen in Öffnungen oder Hohlräumen und vermindert daher auch die Infektionsgefahr für intubierte Patienten.

Günstigerweise kann die Dichtungsmanschette zylinderförmig ausgebildet sein. Eine im Wesentlichen zylinderförmig ausgebildete Dichtungsmanschette weist bei typischen Abmessungen gegenüber den ringförmigen Stirnflächen zwischen dem Beatmungsschlauch und der Mantelfläche eine verhältnismäßig große Manteldichtungsfläche auf, da sich bei einer zylinderförmigen Dichtungsmanschette ein größerer Teil der Außenfläche an die Wand der Trachea anlegen kann als dies bei sonst üblichen kugeligen oder ovalen Dichtungsmanschetten möglich ist. Bevorzugt ist dabei die Manteldichtungsfläche im gleichen radialen Abstand zum Beatmungsschlauch angeordnet, so dass der Beatmungsschlauch sich im Wesentlichen mittig durch die Dichtungsmanschette erstreckt. Durch den gleichmäßigen Abstand der Manteldichtungsfläche zum Beatmungsschlauch wird nicht nur ein gleichmäßiges Anliegen der Manteldichtungsfläche an der Wand der Trachea sondern auch eine Zentrierung des lungenwärtigen Endes des Beatmungsschlauchs in der Trachea erreicht.

Weiter kann der größte Außendurchmesser der zylinderförmigen Dichtungsmanschette im aufgeblasenen Zustand höchstens 15 % größer als der Außendurchmesser an den Enden der zylindrischen Dichtungsmanschette sein. Da die Dichtungsmanschette aufgrund ihrer funktionalen Wirkungsweise aus einem flexiblen oder elastischen Material sowie mit einer möglichst geringen Wandstärke hergestellt ist, weist die zylinderförmig ausgebildete Dichtungsmanschette zumindest im ungestörten aufgeblasenen Zustand eine leicht tonnenförmige Form auf, bei der die Außendurchmesser an den beiden Enden kleiner sind als der üblicherweise in der Mitte der Tonne anzutreffende größte Außendurchmesser. Die geringe Abweichung zwischen dem größten Außendurchmesser und den stirnseitigen Außendurchmessern ermöglicht auch in Längsrichtung der Dichtungsmanschette eine möglichst gleichmäßige Druckverteilung.

Eine bevorzugte Ausgestaltung sieht vor, dass die Wandstärke der Dichtungsmanschette zwischen 0,08 mm und 0,12 mm beträgt. Eine Dichtungsmanschette aus einem flexiblen Material mit einer derart geringen Wandstärke bläst sich bei der Inspiration durch den geringen Eigenwiderstand leicht auf und fällt bei der Exspiration ebenso wieder leicht in sich zusammen.

Eine Variante sieht vor, dass der Abstand zwischen dem lungenwärtigen Ende der Dichtungsmanschette und dem lungenwärtigen Ende des Beatmungsschlauchs kleiner als der zweifache, bevorzugt kleiner als der einfache Außendurchmesser des Beatmungsschlauchs ist. Eine derart nahe Anordnung der Dichtungsmanschette an dem lungenwärtigen Ende des Beatmungsschlauchs vermeidet einen Kontakt des Beatmungsschlauchs mit der Trachea lungenwärts der Dichtungsmanschette und damit auch ungehindertes Einströmen des respiratorischen Gases bei der Inspiration.

Eine besondere Ausführungsform sieht vor, dass die mindestens eine Belüftungskanüle zumindest in dem in die Trachea einführbaren Teil parallel zu und/oder integriert im Beatmungsschlauch angeordnet ist. Eine derartige Fixierung der Belüftungskanüle vermeidet das Vorhandensein von sich gegenseitig störenden Leitungen bzw. reduziert die Anzahl der in die Trachea einzuführenden Leitungen, so dass sowohl das Intubieren selbst als auch das Vorhandensein eines Trachealtubus in der Trachea für den Patienten weniger belastend ist und störende Einflüsse vermieden werden.

Des Weiteren kann der Innendurchmesser der mindestens einen Belüftungskanüle mindestens 20 %, bevorzugt mindestens 25 % des Innendurchmessers des Beatmungsschlauchs betragen. Bei zwei oder mehr Belüftungskanülen ist als Innendurchmesser der äquivalente Innendurchmesser der zwei oder mehr Belüftungskanülen anzusehen. Bei einem derartigen Innendurchmesserverhältnis der Belüftungskanüle ist der Strömungswiderstand in der Kanüle soweit reduziert, dass ein raschen Aufblähen der Dichtungsmanschette bei der Inspiration sowie ein Entweichen der Luft aus bzw. ein Zusammenfallen der Dichtungsmanschette bei der Exspiration möglich ist. In absoluten Werten kann dabei der Innendurchmesser der Belüftungskanüle größer als 1,5 mm, bevorzugt größer als 2,0 mm sein. Dieser absolute Innendurchmesser der Belüftungskanüle bzw. äquivalente Durchmesser mehrerer Kanülen erlaubt bei üblichen Größen des Beatmungsschlauchs eine sichere Funktion der Dichtungsmanschette.

Zur weiteren Reduzierung des Strömungswiderstands der Belüftungskanäle kann die Länge der mindestens einen Belüftungskanüle kleiner als das 1,2-fache, bevorzugt kleiner als das 1,1-fache der Gesamtlänge des Beatmungsschlauchs sein. Ebenso wie ein großer Innendurchmesser ist eine geringe Länge der Belüftungskanüle wichtig, um den Strömungswiderstand zu minimieren und dadurch das Aufblähen und Zusammenfallen der Dichtungsmanschette zu optimieren. Darüber hinaus können sich auch Einschnürungen und Anschlüsse negativ auf den Strömungswiderstand der Belüftungskanüle auswirken und sollten daher vermieden werden.

Die vorliegende Erfindung betrifft weiter die Verwendung eines Trachealtubus zur Beatmung von Patienten mittels eines druckgesteuerten Respirators, mit einem Beatmungsschlauch, einer Dichtungsmanschette und mindestens einer Belüftungskanüle, zum Verbinden mit einem Sprachventil. Der Beatmungsschlauch ist dabei zumindest teilweise in die Trachea einführbar, wobei die Dichtungsmanschette die an dem in die Trachea einführbaren Teil um den Beatmungsschlauch herum angeordnet ist. Die mindestens eine Belüftungskanüle mündet an einem Ende in die Dichtungsmanschette und an seinem der Dichtungsmanschette abgewandten distalen Ende in den Beatmungsschlauch. Die Manteldichtungsfläche der Dichtungsmanschette ist dabei größer als die lungenwärtige ringförmige Stirnfläche der Dichtungsmanschette. Die mindestens eine Belüftungskanüle ist dabei derart ausgebildet, dass diese bei mit dem Beatmungsschlauch verbundenem Sprachventil an dem Sprachventil vorbeigeführt ist und auf der der Dichtungsmanschette abgewandten Seite des Sprachventils in den Beatmungsschlauch mündet. Beim Inspirieren bläst sich die Dichtungsmanschette auf und im aufgeblasenen Zustand liegt eine Manteldichtungsfläche der Dichtungsmanschette an der Trachea an, wobei die Manteldichtungsfläche im aufgeblasenen Zustand größer als die lungenwärtige ringförmige Stirnfläche der Dichtungsmanschette ist. Beim Exspirieren fällt die Dichtungsmanschette zumindest teilweise in sich zusammen und gibt einen Durchgang zwischen der Trachea der Dichtungsmanschette frei, während das Sprachventil eine Rückströmung der Atemluft blockiert. Dies ermöglicht eine zumindest teilweise orale Exspiration, so dass die Atemluft an den Stimmbändern vorbeiströmt und es dem intubierten Patienten ermöglicht zu sprechen.

Die erfindungsgemäße Verwendung eines Trachealtubus ermöglicht ein Ventilieren oder Pulsieren der Dichtungsmanschette im Rhythmus der druckgesteuerten Beatmung, so dass bei der Exspiration des respiratorischen Gases zumindest ein Teil des Atemstroms an der Dichtungsmanschette vorbei oralwärts ausgeatmet wird. Zusammen mit dem oralwärts gerichteten Teil-Atemluftstrom kann auch bronchiales Sekret an der Dichtungsmanschette vorbei oralwärts transportiert werden. Darüber hinaus ermöglich der an den Stimmbändern vorbei oralwärts strömende Teil-Atemstrom eine Stimmerzeugung. Dabei ist es vorteilhaft, wenn die Dichtungsmanschette und die mindestens eine Belüftungskanüle optimal aufeinander abgestimmt und an die Verwendung des Trachealtubus zur Beatmung von Patienten mittels eines druckgesteuerten Respirators angepasst sind, so dass sich der Druck und das Volumen der Dichtungsmanschette bei der Inspiration schnell aufbauen, um die gesamte Lunge des Patienten mit respiratorischem Gas zu füllen, und bei der Exspiration schnell abbauen kann, damit der Teil-Atemluftstrom an der Dichtungsmanschette vorbeigeführt wird, um bronchiales Sekret oralwärts zu transportieren und eine Stimmerzeugung an den Stimmbändern zu ermöglichen.

Im Folgenden werden der Aufbau und die Funktionsweise zweier Ausführungsformen der vorliegenden Erfindung anhand einer Zeichnung näher erläutert.

Es zeigen:
- Fig. 1: einen erfindungsgemäßen Endotrachealtubus mit einer in der Trachea ange- ordneten Dichtungsmanschette,
- Fig. 2: einen erfindungsgemäßen Trachealtubus zur tracheostomatischen Anwen- dung mit einer in der Trachea angeordneten Dichtungsmanschette,
- Fig. 3: die Dichtungsmanschette aus den Fig. 1 und 2 im aufgeblähten Zustand,
- Fig. 4: die in der Trachea angeordnete Dichtungsmanschette aus den Fig. 1 und 2 während der Inspiration, und
- Fig. 5: die in der Trachea angeordnete Dichtungsmanschette aus den Fig. 1 und 2 während der Exspiration.

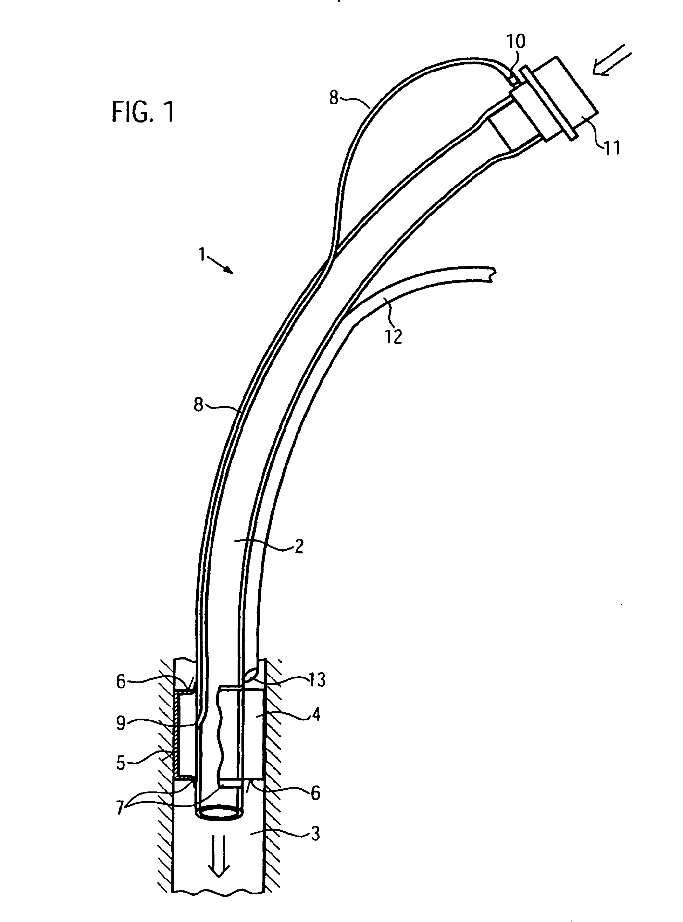

Eine Ausführungsform eines erfindungsgemäßen Trachealtubus 1 zur endotrachealen Anwendung wird in Fig. 1 in einer Seitenansicht dargestellt. Der Trachealtubus 1 umfasst einen biegsamen Beatmungsschlauch 2, der in bekannter Weise in die Trachea (Luftröhre) eingeschoben ist und an seinem oberen Ende mit einem nicht dargestellten druckgesteuerten Respirator (Beatmungsgerät) verbunden ist, das zum periodischen Einführen von respiratorischem Gas in die Lungen dient. Der Beatmungsschlauch 2 ist an seinem lungenwärtigen, in die Trachea 3 eingeführten Ende, mit einer Dichtungsmanschette 4 versehen. Die zylinder- oder tonnenförmige Dichtungsmanschette 4 ist aus einem dünnen elastischen Material gefertigt und weist bei einer Länge von etwa 3,5 cm in einem aufgeblasenen, aber ungespannten und nicht überdehnten Zustand einen Durchmesser von etwa 3 cm auf. Die sich gegenüberliegenden stirnseitigen Enden der Dichtungsmanschette weisen wegen der Flexibilität des elastischen Materials einen minimal geringeren Durchmesser als der mittlere Teil der Dichtungsmanschette 4 auf. In Fig. 1 ist ein Teil der Dichtungsmanschette 4 weggeschnitten, so dass sowohl die zylindrische Form, die dünne Wandstärke als auch das Anliegen der äußeren Manteldichtungsfläche 5 an der Wand der Trachea 3 gut zu erkennen ist. Die Stirnseiten 6 der Dichtungsmanschette 4 sind mittels eines sich in Richtung des Beatmungsschlauches 2 erstreckenden Überstand 7 mit dem Beatmungsschlauch 2 luftdicht verbunden, so dass die Innenfläche der hohlen zylindrischen Dichtungsmanschette von der Außenwandung des Beatmungsschlauches 2 ausgebildet ist. Da die Wandstärke der Dichtungsmanschette 4 sowohl im Bereich des Überstands 7 als auch der Stirnseiten 6 und der Manteldichtungsfläche 5 mit etwa 0,1 mm sehr gering ist, sind auch die im Bereich des Überstands 7 durch die Verbindung mit dem Beatmungsschlauch 2 vergrößerten Schlauchdurchmesser vernachlässigbar. Die Stirnseite 6 der Dichtungsmanschette 4 ist nur ca. 1 cm von dem lungenwärtigen Ende des Beatmungsschlauches 2 entfernt angeordnet, was in etwa dem Außendurchmesser des Beatmungsschlauches 2 entspricht.

Die Dichtungsmanschette 4 wird mittels einer Belüftungskanüle 8 mit Luft aus dem Beatmungsschlauch 2 versorgt. Dabei verläuft die Belüftungskanüle 8 in dem in die Trachea 3 einführbaren Teil des Beatmungsschlauches 2 innerhalb der Wandung des Beatmungsschlauches 2 und mündet mit ihrer Öffnung 9 in die Dichtungsmanschette 4. An dem der Dichtungsmanschette 4 abgewandten distalen Ende 10 mündet die Belüftungskanüle 8 über ein Anschlussstück 11 in den Beatmungsschlauch 2. Das Anschlussstück 11 weist neben dem Stutzen für die Belüftungskanüle 8 auch einen Stutzen auf, an der Beatmungsschlauch 2 angeordnet ist, sowie ein Anschlussende, das mit einem druckgesteuerten Respirator (nicht gezeigt) verbunden wird.

Parallel zu dem in die Trachea 3 einführbaren Teil des Beatmungsschlauches 2 verläuft eine Absaugkanüle 12 bis in den subglottischen Bereich der Trachea 3 oralwärts der Dichtungsmanschette 4. Mittels dieser Absaugkanüle 12 kann über die Absaugöffnung 13 im subglottischen Bereich Sekret und andere Flüssigkeiten aus der Trachea 3 abgesaugt werden. Die Absaugkanüle 12 kann sowohl fest mit dem Beatmungsschlauch 2 verbunden, bzw. darin integriert sein, oder lediglich parallel zum Beatmungsschlauch 2 in die Trachea 3 eingeführt sein.

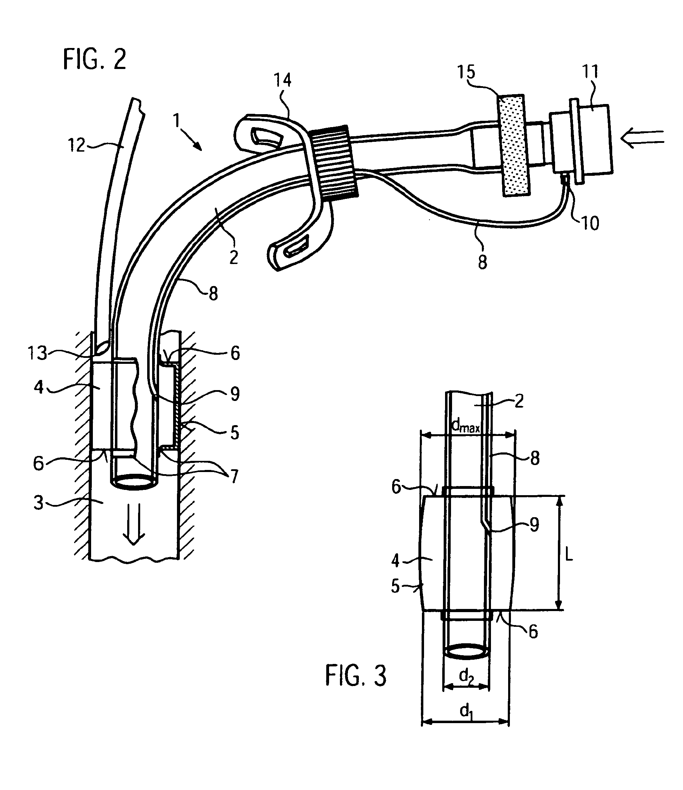

Eine weitere Ausführungsform eines erfindungsgemäßen Trachealtubus 1 zeigt die Fig. 2. Dieser Trachealtubus 1 ist für eine tracheostomatische Anwendung ausgebildet und wird nicht oral sondern über ein Tracheostoma in die Trachea 3 eines Patienten eingeführt. Der Aufbau und die Funktionsweise der an dem lungenwärtigen Ende des Beatmungsschlauches 2 angeordneten Dichtungsmanschette 4 entspricht dem oben für den Endotrachealtubus 1 aus Fig. 1 beschriebenen Dichtungsmanschette 4. Ebenso ist die Anordnung der Belüftungskanüle 8 zum Beatmungsschlauch 2 sowie der Öffnung 9 und der Anschluss des distalen Endes 10 am Anschlussstück 11 mit dem für den Endotrachealtubus 1 aus Fig. 1 beschriebenen Konstruktion identisch. Daher wird im folgenden lediglich auf die Unterschiede des tracheostomatisch eingesetzten Trachealtubus 1 aus Fig. 2 eingegangen.

Bei der in Fig. 1 gezeigten Ausführungsform ist die Absaugkanüle 12 unabhängig von dem Trachealtubus 1, der durch ein entsprechendes Tracheostoma in die Trachea 3 eingeführt ist, und wird oralseitig zugeführt. Über die Absaugöffnung 13 der Absaugkanüle 12 kann wiederum Sekret und andere Flüssigkeiten aus dem subglottischen Bereich der Trachea 3 oralwärts der Dichtungsmanschette 4 abgesaugt werden.

Am Übergang zwischen dem in die Trachea 3 einführbaren Teil des Beatmungsschlauches 2 und dem außerhalb des Tracheostomas verbleibenden Teils ist eine Flügelmanschette 14 vorgesehen, die sich auf dem Beatmungsschlauch 2 unter Widerstand verschieben lässt, um die Position des Beatmungsschlauches 2 in der Trachea 3 und gegenüber dem Tracheostoma zu fixieren. Dazu werden die Flügel der Flügelmanschette 14 seitlich des Tracheostomas am Hals des Patienten fixiert. Als weitere Unterstützung zur exakten Positionierung des Beatmungsschlauches 2 in der Trachea 3 ist der Beatmungsschlauch 2 bei tracheostomatischen Tubussen üblicherweise mit einer Metallspirale versehen, die in der Wandung des Beatmungsschlauches 2 eingebettet ist.

Zwischen dem Anschlussstück 11, das den Trachealtubus 1 mit einem entsprechenden druckgesteuerten Respirator (nicht gezeigt) verbindet, und dem Beatmungsschlauch 2 ist ein Sprechventil 15 vorgesehen, mit dem eine Rückströmung der Atemluft bei der Exspiration verhindert wird, so dass die Abluft vollständig oralwärts an der Dichtungsmanschette 4 vorbei ausgeatmet wird. Da dabei die gesamte Atemluft beim Ausatmen an den Stimmbändern vorbeiströmt, ist es dem intubierten Patienten möglich zu sprechen. Das Sprachventil 15 lässt nur bei der Inspiration die zur Lunge strömende Atemluft durch, während das Sprachventil 15 in der Gegenrichtung blockiert ist.

Fig. 3 zeigt die am lungenwärtigen Ende des Beatmungsschlauches 2 angeordnete Dichtungsmanschette 4 außerhalb der Trachea 3. Dabei ist deutlich die zylindrische bzw. leicht tonnenförmige Form der Dichtungsmanschette 4 zu erkennen, wobei der Durchmesser dₘₐₓ in der Mitte der Dichtungsmanschette 4 (in Längsrichtung gesehen) nur geringfügig größer als die Durchmesser d₁ der Stirnseiten 6 ist. Die bei einem Einsatz in der Trachea 3 an den Tracheawänden anliegende Manteldichtungsfläche 5 bestimmt sich annähernd aus der Gleichung AM = d₁ · π · I, wobei I die Länge der Dichtungsmanschette 4 ist. Während der Inspiration liegt diese Manteldichtungsfläche 5 (AM) zur Abdichtung des Beatmungsschlauches 2 an der Wand der Trachea 3 an. Diese Abdichtung wirkt dem Druck, der von der Lunge auf die Stirnseite 6 der Dichtungsmanschette 4 drückt, entgegen. Die Fläche der Stirnseite 6 der Dichtungsmanschette 4 ergibt sich zu A_{S} = π/₄ · (d₁² - d₂²). Da durch den Anschluss der Belüftungskanüle 8 an den Beatmungsschlauch 2 bei der Inspiration in der Dichtungsmanschette 4 und in der Lunge des Patienten im Wesentlichen der gleiche Druck herrscht, muss die Manteldichtungsfläche 5 größer als die Fläche der Stirnseite 6 sein, um die Abdichtung der Dichtungsmanschette 4 gegenüber den Wänden der Trachea 3 sicherzustellen.

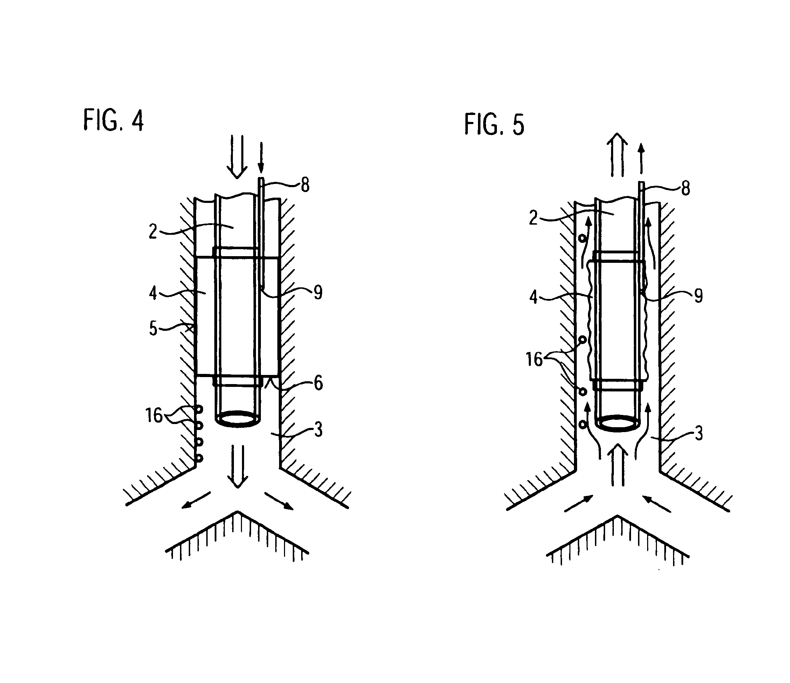

Im Folgenden wird nun die Funktionsweise des erfindungsgemäßen Trachealtubus 1 mit Dichtungsmanschette 4 anhand der Fig. 4 und 5 näher erläutert.

Bei der Inspiration strömt das respiratorische Gas bzw. die Atemluft von dem druckgesteuerten Respirator über den Beatmungsschlauch in die Lunge des Patienten, entsprechend den in den Fig. 1 und 2 sowie 4 dargestellten Pfeilen. Dabei wird gleichzeitig auch über die Belüftungskanüle 8 die Dichtungsmanschette 4 mit Luft gefüllt, so dass die Manteldichtungsfläche 5 an der Wand der Trachea 3 anliegt. Nahezu die gesamte vom Beatmungsgerät erzeugte Atemluft strömt dabei in die Lungen des Patienten. Lediglich ein sehr kleiner Teil der Atemluft wird zum Aufblasen der Dichtungsmanschette 4 verwendet. Im Gegensatz zu den in Fig. 1 und 2 dargestellten Trachealtubussen 1 ist hier die Belüftungskanüle 8 außerhalb des Beatmungsschlauches 2 geführt. Jedoch verläuft die Belüftungskanüle 8 weiterhin parallel zum Beatmungsschlauch und ist zumindest im Bereich der Dichtungsmanschette 4 fest mit dem Beatmungsschlauch 2 verbunden.

Bei der Exspiration reduziert sich zunächst der Druck in der Dichtungsmanschette 4 durch den fehlenden Druck des Beatmungsgeräts, wobei der Druckabbau in der Manschette 4 schneller verläuft als in der Lunge des Patienten. Darüber hinaus strömt zunächst ein Teil der Atemluft aus der Lunge durch den Beatmungsschlauch 2 zurück und unterstützt das Zusammenfallen der Dichtungsmanschette 4 in dem am Einlass der Belüftungskanüle 8 im Anschlussstück 11 ein Unterdruck erzeugt wird und dadurch Luft aus der Dichtungsmanschette abgesaugt wird. Weiter drückt die in der Lunge unter Druck stehende Atemluft gegen die lungenwärtige Stirnseite 6 der Dichtungsmanschette 4 und unterstützt damit ebenfalls das Zusammenfallen oder Teilkollabieren der Dichtungsmanschette 4, wie in Fig. 5 dargestellt, da die Manteldichtungsfläche 5 durch den Druckabfall in der Dichtungsmanschette 4 nicht mehr dichtend an der Wand der Trachea 3 anliegt. Schließlich unterstützt auch der Atemluftstrom das Zusammenfallen der Manschette 4. indem er bei der Exspiration entsprechend der in Fig. 5 gezeigten Pfeile an der Dichtungsmanschette 4 vorbeiströmt.

Das lungenwärts der Dichtungsmanschette 4 in der Trachea 3 vorhandene bronchiale Sekret 16 wird bei der Exspiration durch den an der Dichtungsmanschette 4 vorbeigeführten Teil-Atemluftstrom oralwärts transportiert. Da sich insbesondere bei Tracheostomatubussen oberhalb der Dichtungsmanschette 4 die für den Atemluftstrom zur Verfügung stehende Querschnittsfläche der Trachea 3 wieder vergrößert, reduziert sich die Strömungsgeschwindigkeit der Atemluft und damit auch ihre Fähigkeit, die an der Dichtungsmanschette 4 vorbei mitgerissenen Sekrettropfen 16 weiter oralwärts zu transportieren. Daher kann sich bronchiales Sekret im subglottischen Bereich der Trachea 3 zwischen der Dichtungsmanschette 4 und den Stimmbändern ansammeln und muss zumindest in regelmäßigen Abständen durch eine entsprechende Absaugeinrichtung (nicht gezeigt) entfernt werden.

## Patentansprüche

1. Trachealtubus (1) zur Beatmung von Patienten mittels eines druckgesteuerten Respirators mit einem Beatmungsschlauch (2), der zumindest teilweise in die Trachea (3) einführbar ist, mit einer Dichtungsmanschette (4), die an dem in die Trachea (3) einführbaren Teil um den Beatmungsschlauch (2) herum angeordnet ist, mit mindestens einer Belüftungskanüle (8), die in die Dichtungsmanschette (4) und mit ihrem der Dichtungsmanschette (4) abgewandten Ende (10) in den Beatmungsschlauch (2) mündet, und mit einer Absaugeinrichtung (12), die oralwärts der Dichtungsmanschette (4) absaugend angeordnet ist, wobei die Manteldichtungsfläche (5) der Dichtungsmanschette (4) größer als die lungenwärtige ringförmige Stirnfläche (6) der Dichtungsmanschette (4) ist,
**dadurch gekennzeichnet, dass** ein Sprachventil (15) vorgesehen ist, wobei das Sprachventil an dem nicht in die Trachea (3) einführbaren Teil des Beatmungsschlauchs (2) angeordnet ist und die mindestens eine Belüftungskanüle (8) auf der der Dichtungsmanschette (4) abgewandten Seite des Sprachventils (15) in den Beatmungsschlauch (2) mündet.

2. Trachealtubus (1) nach Anspruch 1,
**dadurch gekennzeichnet, dass** die Innenwand der um den Beatmungsschlauch (2) herum angeordnete Dichtungsmanschette (4) vollständig an dem Beatmungsschlauch (2) anliegt oder von dem Beatmungsschlauch (2) ausgebildet ist.

3. Trachealtubus (1) nach Anspruch 1 oder 2,
**dadurch gekennzeichnet, dass** das Volumen der Dichtungsmanschette (4) zwischen dem Beatmungsschlauch (2) und der Mantelfläche ungeteilt ist.

4. Trachealtubus (1) nach einem der Ansprüche 1 bis 3,
**dadurch gekennzeichnet, dass** die Dichtungsmanschette (4) in dem in die Trachea (3) einführbaren Teil gegenüber dem Beatmungsschlauch (2) geschlossen ausgebildet ist.

5. Trachealtubus (1) nach einem der Ansprüche 1 bis 4,
**dadurch gekennzeichnet, dass** die Dichtungsmanschette (4) zylinderförmig ausgebildet ist.

6. Trachealtubus (1) nach Anspruch 5,
**dadurch gekennzeichnet, dass** der größte Außendurchmesser dₘₐₓ der zylinderförmigen Dichtungsmanschette (4) im aufgeblasenen Zustand höchstens 15 % größer als der Außendurchmesser d₁ an den Enden der zylindrischen Dichtungsmanschette (4) ist.

7. Trachealtubus (1) nach einem der Ansprüche 1 bis 6,
**dadurch gekennzeichnet, dass** die Wandstärke der Dichtungsmanschette (4) zwischen 0,08 mm und 0,12 mm beträgt.

8. Trachealtubus (1) nach einem der Ansprüche 1 bis 7,
**dadurch gekennzeichnet, dass** der Abstand zwischen dem lungenwärtigen Stirnfläche (6) der Dichtungsmanschette (4) und dem lungenwärtigen Ende (10) des Beatmungsschlauchs (2) kleiner als der zweifache, bevorzugt kleiner als der einfache Außendurchmesser des Beatmungsschlauchs (2) ist.

9. Trachealtubus (1) nach einem der Ansprüche 1 bis 8,
**dadurch gekennzeichnet, dass** die mindestens eine Belüftungskanüle (8) zumindest in dem in die Trachea (3) einführbaren Teil parallel zu und/oder integriert im Beatmungsschlauch (2) angeordnet ist.

10. Trachealtubus (1) nach einem der Ansprüche 1 bis 9,
**dadurch gekennzeichnet, dass** der Innendurchmesser der mindestens einen Belüftungskanüle (8) mindestens 20 %, bevorzugt mindestens 25 % des Innendurchmessers des Beatmungsschlauchs (2) beträgt.

11. Trachealtubus (1) nach einem der Ansprüche 1 bis 10,
**dadurch gekennzeichnet, dass** der Innendurchmesser der Belüftungskanüle (8) größer als 1,5 mm, bevorzugt größer als 2,0 mm ist.

12. Trachealtubus (1) nach einem der Ansprüche 1 bis 11,
**dadurch gekennzeichnet, dass** die Länge der mindestens einen Belüftungskanüle (8) kleiner als das 1,2-fache, bevorzugt kleiner als das 1,1-fache der Gesamtlänge des Beatmungsschlauchs (2) ist.

## Claims

1. Tracheal tube (1) for the artificial respiration of patients by means of a pressure-controlled respirator, with a respiratory tube (2), which can be introduced at least partially into the trachea (3), with a sealing sleeve (4), which is arranged around the respiratory tube (2) at the part which can be introduced into the trachea (3), with at least one ventilation cannula (8) which opens into the sealing sleeve (4) and with its end (10) facing away from the sealing sleeve (4) into the respiratory tube (2), and with a suction device (12), which is arranged such as to exert suction in the mouth direction of the sealing sleeve (4), wherein the cover sealing surface (5) of the sealing sleeve (4) is larger than the annular front face surface (6) of the sealing sleeve (4) in the direction of the lung,
**characterised in that** a speech valve (15) is provided, wherein the speech valve is arranged at the part of the respiratory tube (2) which cannot be introduced into the trachea (3), and the at least one ventilation cannula (8) opens into the respiratory tube (2) on the side of the speech valve (15) facing away from the sealing sleeve (4).

2. Tracheal tube (1) according to Claim 1,
**characterised in that** the inner wall of the sealing sleeve (4) arranged around the respiratory tube (2) is in full contact at the respiratory tube (2), or is formed by the respiratory tube (2).

3. Tracheal tube (1) according to Claim 1 or 2,
**characterised in that** the volume of the sealing sleeve (4) between the respiratory tube (2) and the cover surface is undivided.

4. Tracheal tube (1) according to one of Claims 1 to 3,
**characterised in that** the sealing sleeve (4) is formed as closed in the part which can be introduced into the trachea (3) in relation to the respiratory tube (2).

5. Tracheal tube (1) according to one of Claims 1 to 4,
**characterised in that** the sealing sleeve (4) is formed in cylindrical shape.

6. Tracheal tube (1) according to Claim 5,
**characterised in that** the greatest diameter dₘₐₓ of the cylindrical sealing sleeve (4) in the inflated state is at the most 15 % larger than the outer diameter d₁ at the ends of the cylindrical sealing sleeve (4).

7. Tracheal tube (1) according to one of Claims 1 to 6,
**characterised in that** the wall thickness of the sealing sleeve (4) amounts to between 0.08 mm and 0.12 mm.

8. Tracheal tube (1) according to one of Claims 1 to 7,
**characterised in that** the space between the face surface (6) in the direction of the lung of the sealing sleeve (4) and the lung end (10) of the respiratory tube (2) is smaller than twice, and preferably smaller than the single outer diameter of the respiratory tube (2).

9. Tracheal tube (1) according to one of Claims 1 to 8,
**characterised in that** the at least one ventilation cannula (8) is arranged, at least in the part which can be introduced into the trachea (3), parallel to and/or integrated in the respiratory tube (2).

10. Tracheal tube (1) according to one of Claims 1 to 9,
**characterised in that** the inner diameter of the at least one ventilation cannula (8) amounts to at least 20 %, preferably at least 25 %, of the inner diameter of the respiratory tube (2).

11. Tracheal tube (1) according to one of Claims 1 to 10, **characterised in that** the inner diameter of the ventilation cannula (8) is greater than 1.5 mm, preferably greater than 2.0 mm.

12. Tracheal tube (1) according to one of Claims 1 to 11, **characterised in that** the length of the at least one ventilation cannula (8) is 1.2 times, preferably 1.1 times smaller than the total length of the respiratory tube (2).

## Revendications

1. Tube trachéal (1) pour la respiration artificielle pratiquée sur des patients à l'aide d'un respirateur commandé par pression, avec un tuyau de respiration (2) qui est apte à être introduit au moins en partie dans la trachée (3), un manchon d'étanchéité (4) qui est disposé, au niveau de la partie apte à être introduite dans la trachée (3), autour du tuyau de respiration (2), au moins une canule d'aération (8) qui débouche dans le manchon d'étanchéité (4) et, avec son extrémité (10) opposée au manchon d'étanchéité (4), dans le tuyau de respiration (2), et un dispositif d'aspiration (12) qui est dirigé vers la bouche, à partir du manchon d'étanchéité (4), en vue d'une aspiration, étant précisé que la surface d'étanchéité latérale (5) du manchon (4) est plus grande que la surface frontale annulaire (6) du manchon (4) dirigée vers les poumons,
**caractérisé en ce qu'**il est prévu une valve phonatoire (15), ladite valve phonatoire étant disposée au niveau de la partie du tube de respiration (2) qui n'est pas apte à être introduite dans la trachée (3), et la ou les canules d'aération (8) débouchant, du côté de la valve phonatoire (15) opposé au manchon d'étanchéité (4), dans le tuyau de respiration (2).

2. Tube trachéal (1) selon la revendication 1, **caractérisé en ce que** la paroi intérieure du manchon d'étanchéité (4) disposé autour du tuyau de respiration (2) est appliquée entièrement contre le tuyau de respiration (2) ou est formée par le tuyau de respiration (2).

3. Tube trachéal (1) selon la revendication 1 ou 2, **caractérisé en ce que** le volume du manchon d'étanchéité (4), entre le tuyau de respiration (2) et la surface latérale, est non divisé.

4. Tube trachéal (1) selon l'une des revendications 1 à 3, **caractérisé en ce que** le manchon d'étanchéité (4), dans la partie apte à être introduite dans la trachée (3), est conçu pour être fermé par rapport au tuyau de respiration (2).

5. Tube trachéal (1) selon l'une des revendications 1 à 4, **caractérisé en ce que** le manchon d'étanchéité (4) est cylindrique.

6. Tube trachéal (1) selon la revendication 5, **caractérisé en ce que** le diamètre extérieur maximal d_{maX} du manchon d'étanchéité cylindrique (4), à l'état gonflé, est supérieur de 15 % au maximum au diamètre extérieur d₁ aux extrémités du manchon d'étanchéité cylindrique (4).

7. Tube trachéal (1) selon l'une des revendications 1 à 6, **caractérisé en ce que** l'épaisseur de paroi du manchon d'étanchéité (4) est située entre 0,08 mm et 0,12 mm.

8. Tube trachéal (1) selon l'une des revendications 1 à 7, **caractérisé en ce que** la distance entre la surface frontale (6) du manchon d'étanchéité (4) située côté poumons et l'extrémité (10) du tuyau de respiration (2) située côté poumons est inférieure à deux fois, de préférence inférieure à une fois le diamètre extérieur du tuyau de respiration (2).

9. Tube trachéal (1) selon l'une des revendications 1 à 8, **caractérisé en ce que** la ou les canules d'aération (8), au moins dans la partie apte à être introduite dans la trachée (3), sont disposées parallèlement au tuyau de respiration (2) et/ou sont intégrées dans celui-ci.

10. Tube trachéal (1) selon l'une des revendications 1 à 9, **caractérisé en ce que** le diamètre intérieur de la ou des canules d'aération (8) représente au moins 20 %, de préférence au moins 25 % du diamètre intérieur du tuyau de respiration (2).

11. Tube trachéal (1) selon l'une des revendications 1 à 10, **caractérisé en ce que** le diamètre intérieur de la canule d'aération (8) est supérieur à 1,5 mm, de préférence supérieur à 2,0 mm.

12. Tube trachéal (1) selon l'une des revendications 1 à 11, **caractérisé en ce que** la longueur de la ou des canules d'aération (8) est inférieure à 1,2 fois, de préférence inférieure à 1,1 fois la longueur totale du tuyau de respiration (2).
